# EUROPEAN PATENT APPLICATION

(11) **EP 2 647 718 A2**
(43) Date of publication of application: **09.10.2013**
(21) Application number: 13305442.9
(22) Date of filing: 05.04.2013
(51) Int. Cl.: C12P 13/00, C12N 9/10, C12N 9/88

(54) **Process for producing 5-aminopentanoate empolying a recombinant microorganism**

(30) Priority: 06.04.2012 US 201261621404 P
(71) Applicant: Metabolic Explorer, 63360 Saint Beauzire (FR)
(72) Inventor: Letellier, Guillaume, 63530 VOLVIC (FR)
(74) Representative: Regimbeau

(57) **Abstract**

The present invention concerns a method for the biological preparation of 5-aminopentanoate comprising culturing a microorganism genetically modified for the bioproduction of 5-aminopentanoate, wherein the microorganism comprises a metabolic pathway for conversion of lysine or 2-oxoadipate into 5-aminopentanoate.

## Description

The present invention concerns a method for the preparation of 5-aminopentanoate, in recombinant microorganisms and in particular in recombinant yeasts.

### INTRODUCTION

5-aminopentanoate has the following formula:

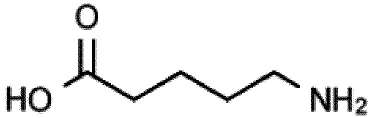

### PRIOR ART

Although the main industrial way for producing 5-aminopentanoate is chemical synthesis, some biosynthetic pathways have been identified recently.

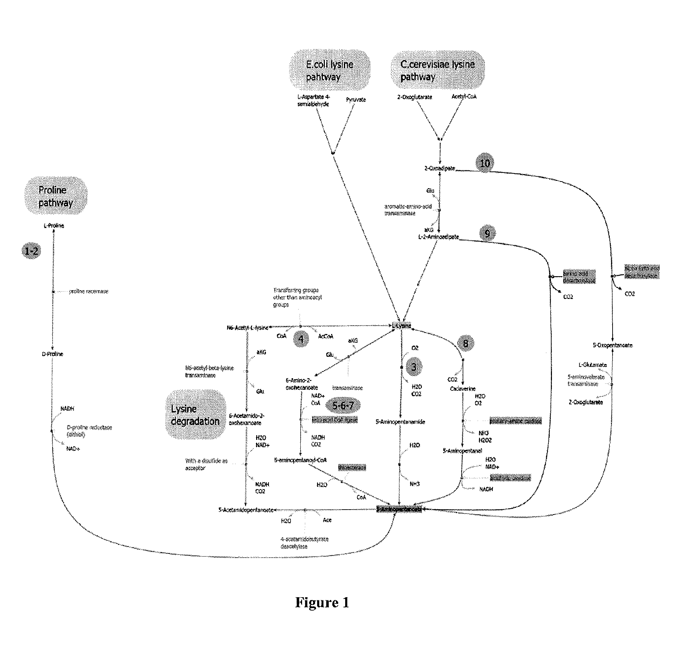

The patent application WO 2010/068953 provides a method for producing 5-aminopentanoate (5AP) with recombinant host cells, based on the degradation pathway of lysine (see figure 1 - pathways N°1, 2, 3).

### DESCRIPTION OF THE INVENTION

The present invention is related to new biosynthesis pathways for 5-aminopentanoate in a recombinant microorganism.

### DRAWINGS

**Figure 1****.** Ten metabolic pathways for biosynthesis of 5-aminopentanoate.

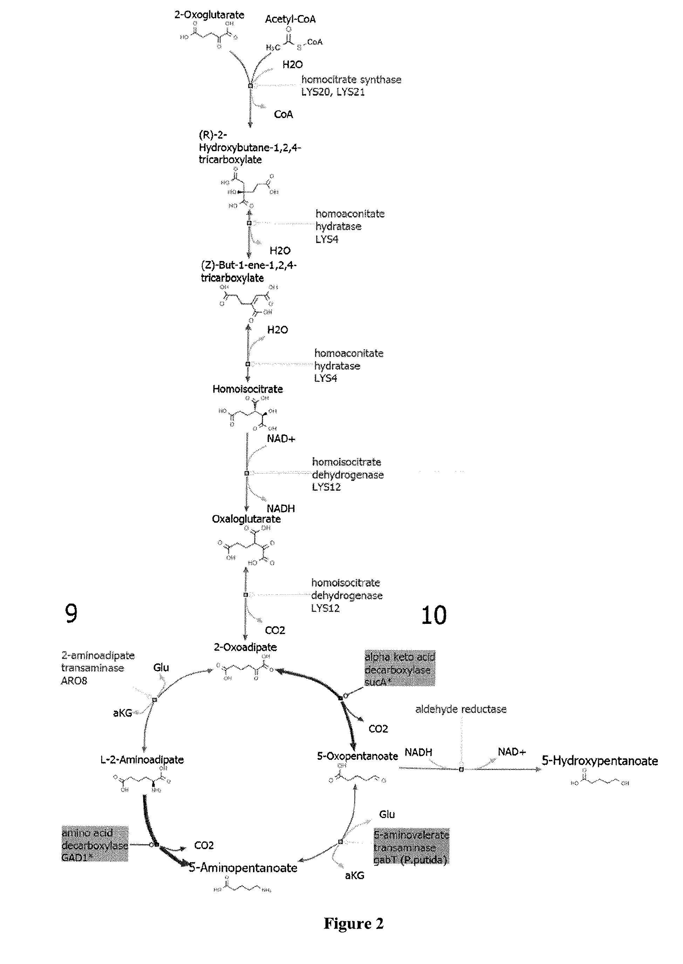

**Figure 2****.** Two metabolic pathways for biosynthesis of 5-aminopentanoate.

### DETAILED DESCRIPTION OF THE INVENTION

Before describing the present invention in detail, it is to be understood that this invention is not limited to particularly exemplified methods and may, of course, vary. It is also to be understood that the terminology used herein is for the purpose of describing particular embodiments of the invention only, and is not intended to be limiting, which will be limited only by the appended claims.

All publications, patents and patent applications cited herein, whether *supra* or *infra,* are hereby incorporated by reference in their entirety. However, publications mentioned herein are cited for the purpose of describing and disclosing the protocols, reagents and vectors that are reported in the publications and that might be used in connection with the invention. Nothing herein is to be construed as an admission that the invention is not entitled to antedate such disclosure by virtue of prior invention.

Furthermore, the practice of the present invention employs, unless otherwise indicated, conventional microbiological and molecular biological techniques within the skill of the art. Such techniques are well known to the skilled worker, and are explained fully in the literature. See, for example, Guthrie & Fink, 2004 and Sambrook *et al*., (1999) (2001).

It must be noted that as used herein and in the appended claims, the singular forms "a", "an", and "the" include plural reference unless the context clearly dictates otherwise. Thus, for example, a reference to "a microorganism" includes a plurality of such microorganisms, and a reference to "an endogenous gene" is a reference to one or more endogenous genes, and so forth. Unless defined otherwise, all technical and scientific terms used herein have the same meanings as commonly understood by one of ordinary skill in the art to which this invention belongs. Although any materials and methods similar or equivalent to those described herein can be used to practice or test the present invention, the preferred materials and methods are now described.

In the claims that follow and in the description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

### Definitions

The term "5-aminopentanoate" as used herein refers to a chemical compound having the formula C5H11N02. Common synonyms are : 5-Aminopentanoate, 5-Aminopentanoic acid, 5-Aminovalerate, 5-Aminovaleric acid, delta-Amino-n-valerate, delta-Amino-n-valeric acid, delta-Aminovalerate, delta-Aminovaleric acid.

The terms "activity" and "function" refer to a specific catalytic activity or function of an enzyme, i.e. the biochemical reaction(s) that is(are) catalyzed by this enzyme.

The term "microorganism", as used herein, refers to a bacterium, yeast or fungus which is not modified artificially.

The term "recombinant microorganism" or "genetically modified microorganism", as used herein, refers to a microorganism genetically modified or genetically engineered. It means, according to the usual meaning of these terms, that the microorganism of the invention is not found in nature and is modified either by introduction, by deletion or by modification of genetic elements.

A microorganism may be modified to express exogenous genes if these genes are introduced into the microorganism with all the elements allowing their expression in the host microorganism. A microorganism may be modified to modulate the expression level of an endogenous gene. The modification or "transformation" of microorganisms with exogenous DNA is a routine task for those skilled in the art.

The 'activity' of an enzyme is used interchangeably with the term 'function' and designates, in the context of the invention, the reaction that is catalyzed by the enzyme.

The terms "encoding" or "coding" refer to the process by which a polynucleotide, through the mechanisms of transcription and translation, produces an amino-acid sequence.

In a specific embodiment of the invention, at least one of the enzymes involved in the 5-aminopentanoate biosynthesis pathway is an evolved enzyme.

This term 'evolved' designates enzymes that possess at least one mutation in their sequence, in comparison with the aminoacid sequence of the wild-type enzyme, said mutation leading to an increase of their activity or their specificity for the substrate. These enzymes have been selected in function of their improved activity or specificity for the substrate. In particular, these enzymes can be specific for a substrate in their wild-type state, and become specific for another substrate after the mutation(s) occurs.

Evolved enzymes can be obtained according to all techniques well known by the man skilled in the art; all evolved enzymes are tested for their activity and substrate specificity in a 'test' microorganism; the enzymes showing an improved activity and/or specificity for the substrate are identified and isolated, and preferentially are sequenced to identify the existing mutations. The genes encoding these identified evolved enzymes are cloned into a vector and introduced into the microorganism according to the invention, this recombinant microorganism being used for the fermentative preparation of 5-aminopentanoate.

All techniques for transforming the microorganism, in particular to introduce expression vectors, and regulatory elements used for enhancing the production of 5-aminopentanoate, are well known in the art and available in the literature, including the applicant's own patent applications on the modification of biosynthesis pathways in various microorganisms, including WO 2008/052973, WO 2008/052595, WO 2008/040387, WO 2007/144346, WO 2007/141316, WO 2007/077041, WO 2007/017710, WO 2006/082254, WO 2006/082252, WO 2005/111202, WO 2005/073364, WO 2005/047498, WO 2004/076659, the content of which is incorporated herein by reference.

Expression vectors carrying genes encoding the evolved enzymes allow an overexpression of said genes and corresponding encoded enzymes. The term 'overexpression' means in this context that the expression of the gene or of the enzyme is increased compared to a non modified microorganism.

The introduced genes can be heterologous or not. The term "heterologous gene" means that the gene is derived from a species of microorganism different from the recipient microorganism that expresses it. It refers to a gene which is not naturally occurring in the microorganism. A microorganism can express exogenous genes if these genes are introduced into the microorganism with all the elements allowing their expression in the host microorganism. Transforming microorganisms with exogenous DNA is a routine task for the man skilled in the art. These introduced genes may be integrated into the host chromosome, or be expressed extra-chromosomally by plasmids or vectors. A variety of plasmids, which differ with respect to their origin of replication and their copy number in the cell, are all known in the art.

In the present application, all genes are referenced with their common names and with references that give access to their nucleotidic sequences on the website http://www.ncbi.nlm.nih.gov/gene.

The sequence of exogenous genes may be adapted for its expression in the host microorganism. Indeed, the man skilled in the art knows the notion of codon usage bias and how adapt nucleic sequence for a particular codon usage bias without modify the deduced protein.

In another embodiment of the invention, other genetic modifications can be performed in the recombinant microorganism. In particular the expression of some genes can be attenuated. "Attenuation" of genes may be achieved by means and methods known to the man skilled in the art and contains gene deletion by homologous recombination, gene attenuation by insertion of an external element into the gene or gene expression under a weak promoter. The man skilled in the art knows a variety of promoters which exhibit different strength and which promoter to use for a weak genetic expression.

The "fermentation" is generally conducted in fermenters with an appropriate culture medium adapted to the microorganism being used, containing at least one simple carbon source, and if necessary co-substrates.

An "culture medium" designates a medium (e.g., a sterile, liquid media) comprising nutrients essential or beneficial to the maintenance and/or growth of the cell such as carbon sources or carbon substrate, nitrogen sources, for example, peptone, yeast extracts, meat extracts, malt extracts, urea, ammonium sulfate, ammonium chloride, ammonium nitrate and ammonium phosphate; phosphorus sources, for example, monopotassium phosphate or dipotassium phosphate; trace elements (e.g., metal salts), for example magnesium salts, cobalt salts and/or manganese salts; as well as growth factors such as amino acids, vitamins, growth promoters, and the like.

The term "carbon source" or "carbon substrate" or "source of carbon" according to the present invention denotes any source of carbon that can be used by those skilled in the art to support the normal growth of a yeast, including hexoses (such as glucose, galactose or lactose), pentoses, monosaccharides, oligosaccharides, disaccharides (such as sucrose, cellobiose or maltose), molasses, starch or its derivatives, cellulose, hemicelluloses and combinations thereof.

In some embodiments of the invention, the carbon source is a by-product of another process using biomass as starting material, or eventually, the product of mechanical and/or chemical and/or enzymatic degradation of biomass, such as degradation of cellulose.

Other carbon substrates may include ethanol, lactate, succinate, or glycerol. Hence it is contemplated that the source of carbon utilized in the present invention may encompass a wide variety of carbon containing substrates and will only be limited by the choice of organism. In a specific embodiment of the invention, the substrate may be hydrolysate of second generation carbons sources such wheat straw, miscanthus plants, sugar cane bagasses, wood and others that are known to the man skilled in the art.

Although it is contemplated that all of the above mentioned carbon substrates and mixtures thereof are suitable in the present invention, preferred carbon substrates are glucose, fructose, and sucrose, or mixtures of these with C5 sugars such as xylose and/or arabinose for yeasts cells modified to use C5 sugars.

In addition to an appropriate carbon source, fermentation media must contain suitable minerals, salts, cofactors, buffers and other components, known to those skilled in the art, suitable for the growth of the cultures and promotion of the enzymatic pathway necessary for the production of the desired product.

As an example of known culture media for *E*. *coli,* the culture medium can be of identical or similar composition to an M9 medium (Anderson, 1946), an M63 medium (Miller, 1992) or a medium such as defined by Schaefer *et al.,* (1999).

Those skilled in the art are able to define the culture conditions for the microorganisms according to the invention. In particular the bacteria are fermented at a temperature between 20°C and 55°C, preferentially between 25°C and 40°C, and more specifically about 37°C for *E*. *coli.*

Typically, yeast cells are grown at a temperature in the range of about 20 °C to about 37 °C in an appropriate medium. Suitable growth media in the present invention are common commercially prepared media such as broth that includes yeast nitrogen base, ammonium sulfate, and dextrose as the carbon/energy source) or YPD Medium, a blend of peptone, yeast extract, and dextrose in optimal proportions for growing most

Other defined or synthetic growth media may also be used and the appropriate medium for growth of the particular microorganism will be known by one skilled in the art of microbiology or fermentation science.

Suitable pH ranges for the fermentation are between pH 3.0 to pH 7.5, where pH 4.5 to pH 6.5 is preferred as the initial condition.

Fermentations may be performed under aerobic or anaerobic conditions, where anaerobic or micro-aerobic conditions are preferred.

The terms "anaerobic conditions" refer to conditions under which the oxygen concentration in the culture medium is too low for the microorganism to be used as terminal electron acceptor. Anaerobic conditions may be achieved either by sparging the culture medium with an inert gas such as nitrogen until oxygen is no longer available to the microorganism or by the consumption of the available oxygen by the microorganism.

"Aerobic conditions" refers to concentrations of oxygen in the culture medium that are sufficient for an aerobic or facultative anaerobic microorganism to use as a terminal electron acceptor.

The amount of product in the fermentation medium can be determined using a number of methods known in the art, for example, high performance liquid chromatography (HPLC) or gas chromatography (GC).

The present process may employ a batch method of fermentation. A classical batch fermentation is a closed system where the composition of the medium is set at the beginning of the fermentation and not subject to artificial alterations during the fermentation. Thus, at the beginning of the fermentation the medium is inoculated with the desired organism or organisms, and fermentation is permitted to occur without adding anything to the system. Typically, however, a "batch" fermentation is batch with respect to the addition of carbon source and attempts are often made at controlling factors such as pH and oxygen concentration. In batch systems the metabolite and biomass compositions of the system change constantly up to the time when the fermentation is stopped. Within batch cultures cells progress through a static lag phase to a high growth log phase and finally to a stationary phase where growth rate is diminished or halted. If untreated, cells in the stationary phase will eventually die. Cells in log phase generally are responsible for the bulk of production of end product or intermediate.

A Fed-Batch system may also be used in the present invention. A Fed-Batch system is similar to a typical batch system with the exception that the carbon source substrate is added in increments as the fermentation progresses. Fed-Batch systems are useful when catabolite repression (e.g. glucose repression) is apt to inhibit the metabolism of the cells and where it is desirable to have limited amounts of substrate in the media. Measurement of the actual substrate concentration in Fed-Batch systems is difficult and is therefore estimated on the basis of the changes of measurable factors such as pH, dissolved oxygen and the partial pressure of waste gases such as CO₂.

Fermentations are common and well known in the art and examples may be found in Sunderland *et al.,* (1992), herein incorporated by reference. Although the present invention is performed in batch mode it is contemplated that the method would be adaptable to continuous fermentation.

Continuous fermentation is an open system where a defined fermentation medium is added continuously to a bioreactor and an equal amount of conditioned media is removed simultaneously for processing. Continuous fermentation generally maintains the cultures at a constant high density where cells are primarily in log phase growth.

Continuous fermentation allows for the modulation of one factor or any number of factors that affect cell growth or end product concentration. For example, one method will maintain a limiting nutrient such as the carbon source or nitrogen level at a fixed rate and allow all other parameters to vary. In other systems a number of factors affecting growth can be altered continuously while the cell concentration, measured by media turbidity, is kept constant. Continuous systems strive to maintain steady state growth conditions and thus the cell loss due to the medium being drawn off must be balanced against the cell growth rate in the fermentation. Methods of modulating nutrients and growth factors for continuous fermentation processes as well as techniques for maximizing the rate of product formation are well known in the art of industrial microbiology.

It is contemplated that the present invention may be practiced using either batch, fed-batch or continuous processes and that any known mode of fermentation would be suitable. Additionally, it is contemplated that cells may be immobilized on a substrate as whole cell catalysts and subjected to fermentation conditions for production.

The present invention is related to a method for the fermentative production of **5-aminopentanoate**, via different biosynthesis pathways. Ten biosynthesis pathways are represented in figure 1; four of these biosynthesis pathways are described extensively below.

According to the invention, all enzymes cited in the present application are issued from the species as specified, or are similar enzymes from different species, having the same enzymatic activity i.e. being functional equivalents. These similar enzymes can be easily identified by the man skilled in the art, for example with the databases EXPASY, or Proteomics, or BLAST, accessible online. Genes encoding these enzymes can also be identified easily with, for example, the database UNIPROT, accessible online.

### 1. 5-aminopentanoate biosynthesis by fungal lysine biosynthesis pathway : pathway N°9

In this embodiment of the invention, the 5-aminopentanoate biosynthesis pathway comprises the following successive steps:
- Conversion of 2-oxoadipate into L-2-aminoadipate by the catalytic action of an amino acid transaminase;
- Conversion of said L-2-aminoadipate into -aminopentanoate by the catalytic action of an evolved amino acid decarboxylase.

In a particular embodiment of the invention, the amino acid transaminase is an aromatic amino acid transaminase; more preferably, it is encoded by the gene *ARO8* from S.cerevisiae.

The enzyme having "aminoacid decarboxylase" activity is a decarboxylase acting specifically on amino acids.

In a particular embodiment of the invention, the aminoacid decarboxylase activity is performed with an evolved glutamate decarboxylase (EC:4.1.1.15), that naturally catalyzes the following reaction:

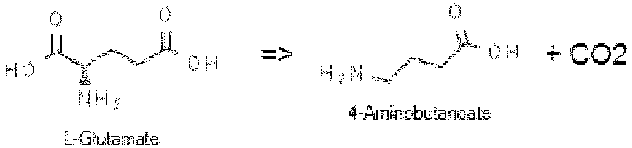

Genes encoding this enzyme are, for example : *GAD4* from *A.thaliana*; *GAD5* from *A.thaliana*; *GAD* from *A.thaliana*; *GAD3* from *A.thaliana*; *GAD2* from *A.thaliana*; *GAD1* from *C.albicans*; *unc-25* from *C.elegans*; *gadA* from *E.coli*; *gadB* from *E.coli*; *GAD1* from *S.cerevisiae*; *gad* from *S.coelicolor.*

In another embodiment of the invention, the aminoacid decarboxylase activity is performed with an evolved diaminopimelate decarboxylase (EC 4.1.1.20) that naturally catalyzes the following reaction:

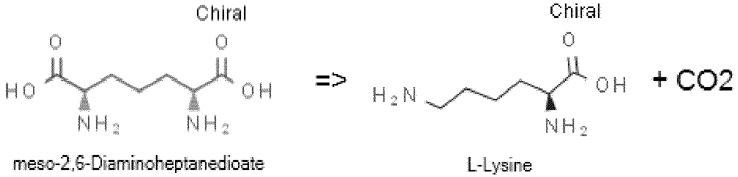

Genes encoding this enzyme are, for example:
*lysA; B.pertussis*
*lysA; B.subtilis*
*lisA; C.acetobutylicum*
*cgl1180; C.glutamicum*
*cgl2135; C.glutamicum*
*lysA; E.coli*
*lysA; L.lactis*
*lysA; S. coelicolor*

In another embodiment of the invention, the aminoacid decarboxylase activity is performed with an evolved aspartate decarboxylase (EC 4.1.1.11), that naturally catalyzes the following reaction:

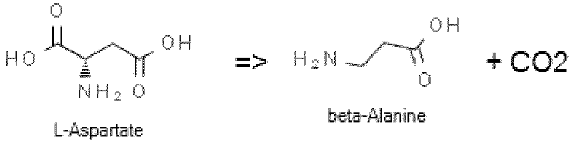

Genes encoding this enzyme are, for example:

| | |
|---|---|
| *panD* | *B.cereus_03BB102* |
| *cgl0135* | *C.glutamicum* |
| *panD* | *C.jejuni* |
| *panD* | *E.coli* |
| *panD* | *S.coelicolor* |

In another embodiment of the invention, the aminoacid decarboxylase activity is performed with an evolved Ornithine decarboxylase (4.1.1.17), that naturally catalyzes the following reaction:

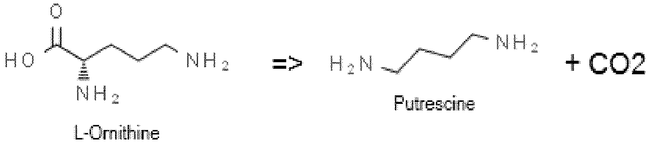

Genes encoding this enzyme are, for example:

| | |
|---|---|
| *speC* | *E.coli* |
| *speF* | *E.coli* |
| *speC* | *P.aeruginosa* |
| *ypch01283* | *R.etli* |
| *speF* | *L.casei* |
| *SPE1* | *S.cerevisiae* |
| *Adc* | *M.musculus* |
| *Azin1* | *M.musculus* |
| *adc* | *X.laevis* |

In a prefered embodiment of the invention, the evolved amino acid decarboxylase is encoded by a mutated gene *GAD1 from S. cerevisiae.* The glutamate decarboxylase encoded by *GAD1* is not naturally specific for the substrate '2-aminoadipate', but an evolved enzyme having specificity for this substrate is selected from mutated enzymes.

### 2. 5-aminopentanoate biosynthesis by fungal lysine biosynthesis pathway : pathway N°10

In this particular embodiment of the invention, the 5-aminopentanoate biosynthesis pathway comprises the following successive steps:
- Conversion of 2-oxoadipate into 5-oxopentanoate by the catalytic action of an evolved alpha-keto acid decarboxylase: 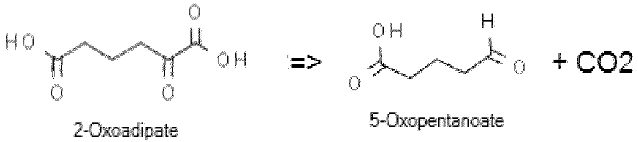
- Conversion of said 5-oxopentanoate into 5-aminopentanoate by the catalytic action of a transaminase.

Decarboxylases catalyse chemical reactions of decarboxylation, that releases carbon dioxide (CO2). In particular, decarboxylation refers to a reaction of carboxylic acids, removing a carboxyl group from a carbon chain. Enzymes that catalyze decarboxylations are called decarboxylases or, the more formal term, carboxy-lyases (EC number 4.1.1.-).

In a particular embodiment, said enzyme is an evolved α-ketoglutarate decarboxylase (EC 4.1.1.71), that catalyzes naturally the following reaction:

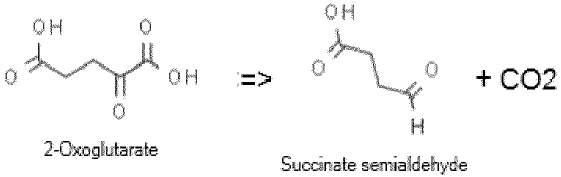

Genes encoding this enzyme are, for example:

| | |
|---|---|
| *menD* | *B.cereus_03BB102* |
| *menD* | *B.subtilis* |
| *menD* | *E.coli* |
| *menD* | *L.lactis* |
| *kgd* | *S.viridis* |
| *menD* | *Synechococcus_CC9311* |

In another particular embodiment, said enzyme is an evolved ketoisovalerate decarboxylase (EC:4.1.1.74), that catalyzes naturally the following reaction:

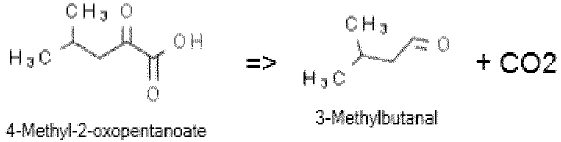

Genes encoding this enzyme are, for example:

| | |
|---|---|
| *KivD* | *L.Lactis* |
| *ilvG* | *E.coli S88* |
| *PDC1* | *S.cerevisiae* |
| *ypf00238* | *R.etli* |
| *ipdC* | *R.eutropha_H16* |

In another particular embodiment, said enzyme is an evolved enzyme issued from *Pseudomonas,* that catalyzes naturally the following reaction:

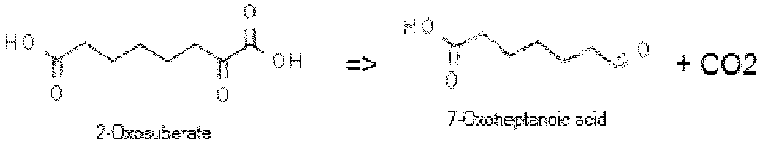

According to a specific embodiment of the invention, the alpha-keto acid decarboxylase is encoded by a gene *sucA* from S.cerevisiae. Preferentially, an evolved enzyme having alpha-keto acid decarboxylase has been chosen, as described previously.

The second reaction of this biosynthesis pathway is catalysed by an enzyme having transaminase activity; preferentially, said enzyme has a "5-aminovalerate transaminase" activity (EC 2.6.1.48), that catalyzes the following chemical reaction, in both ways:
5-oxopentanoate + L-glutamate → 5-aminopentanoate + 2-oxoglutarate

This enzyme belongs to the family of transferases, specifically the transaminases, which transfer nitrogenous groups (EC 2.6.1.-). The systematic name of this enzyme class is 5-aminopentanoate:2-oxoglutarate aminotransferase. Other names in common use include 5-aminovalerate aminotransferase, delta-aminovalerate aminotransferase, and delta-aminovalerate transaminase. This enzyme participates in lysine degradation. It employs one cofactor, pyridoxal phosphate.

Several genes encode enzymes with 5-aminovalerate transaminase activity:

| | |
|---|---|
| *gabT* | *A.vinelandii* |
| *yodT* | *B.amyloliquefaciens_DSM7* |
| *yodT* | *B.subtilis* |
| *gabT* | *P.aeruginosa* |
| *gabT* | *P.entomophila* |
| *gabT* | *P.fluorescens* |
| *gabT* | *P.putida* |
| *gabT* | *P.stutzeri* |
| *gabT-2* | *P.syringae* |

In a particular aspect of the invention, the 5-aminovalerate transaminase is an enzyme encoded by the gene *gabT* from *P. putida.*

### 3. 5-aminopentanoate biosynthesis by E. coli lysine degradation pathways n°5, 6, 7

In this specific aspect of the invention, the 5-aminopentanoate biosynthesis pathway comprises the following successive steps:
- Conversion of lysine into 6-amino-2-oxohexanoate by catalytic action of a transaminase;
- Conversion of 6-amino-2-oxohexanoate into 5-aminopentanoyl-coA by catalytic action of a ketoacid coA ligase;
- Conversion of 5-aminopentanoyl-coA into 5-aminopentanoate by catalytic action of a thioesterase.

### 4. 5-aminopentanoate biosynthesis by E. coli lysine degradation pathway: pathway n°4

In this aspect of the invention, the 5-aminopentanoate biosynthesis pathway comprises the following successives steps:
- Conversion of lysine into N6-acetyl-L-lysine by the catalytic action of a N-acetyl transferase,
- Conversion of N6-acetyl-L-lysine into 6-acetamido-2-oxohexanoate by the catalytic action of a N6-acetyl-beta-lysine transaminase,
- Conversion of 6-acetamido-2-oxohexanoate into 5-acetamidopentanoate, for example by the catalytic action of an alpha keto acid decarboxylase,
- Conversion of 5-acetamidopentanoate into 5-aminopentanoate by the catalytic action of a 4-acetamidobutyrate deacetylase.

Preferentially, the microorganism of the invention is selected among yeast or bacteria.

Bacteria are preferentially chosen among *Enterobacteriaceae, Clostridiaceae, Bacillaceae, Streptomycetaceae,* and *Corynebacteriaceae.* More preferentially the microorganism is a species of *Escherichia, Clostridium, Bacillus, Klebsiella, Pantoea, Salmonella, Pseudomonas, or Corynebacterium.*

Preferentially, the yeast of the invention is selected among genera *Saccharomyces, Candida, Kluyveromyces, Ashbya, Dekkera, Pichia (Hansenula), Debaryomyces, Clavispora, Lodderomyces, Yarrowia, Schizosaccharomyces, Cryptococcus, Malassezia.* More preferentially the yeast is a species of *Saccharomyces, Pichia, Kluyveromyces* or *Yarrowia.* According to a specific aspect of the invention, the yeast is from the species *Saccharomyces cerevisiae* and *Kluyveromyces lactis* or *Kluyveromyces marxianus.*

According to a specific aspect of the invention, the microorganism is from the species *Escherichia coli* or *Saccharomyces cerevisiae.*

According to a specific aspect of the invention, the fermentative production of 5-aminopentanoate comprises a step of isolation of the 5-aminopentanoate from the culture medium. Recovering the 5-aminopentanoate from the culture medium is a routine task for a man skilled in the art. It may be achieved by a number of techniques well known in the art including but not limiting to crystallization, distillation, gas-stripping, pervaporation or liquid extraction. The expert in the field knows how adapt parameters of each technic dependant of the characteristics of the material to be separated.

Crystallization is a chemical solid-liquid separation technique, in which mass transfer of a solute from the liquid solution to a pure solid crystalline phase occurs, in a crystallizer. Preferentially, a preliminary step of micro-filtration or ultra-filtration is performed before the crystallization step.

Distillation may involve an optional component different from the culture medium in order to facilitate the isolation of prenol by forming azeotrope and notably with water. This optional component is an organic solvent such as cyclohexane, pentane, butanol, benzene, toluene, trichloroethylene, octane, diethylether or a mixture thereof.

Gas stripping is achieved with a stripping gas chosen among helium, argon, carbon dioxide, hydrogen, nitrogen or mixture thereof.

Liquid extraction is achieved with organic solvent as the hydrophobe phase such as pentane, hexane, heptane, dodecane.

The present invention is also related to a genetically modified microorganism for the fermentative production of 5-aminopentanoate, as defined above.

The present invention is also related to a method of conversion of lysine into 5-aminopentanoate with enzymes, according to the following scheme of reations:

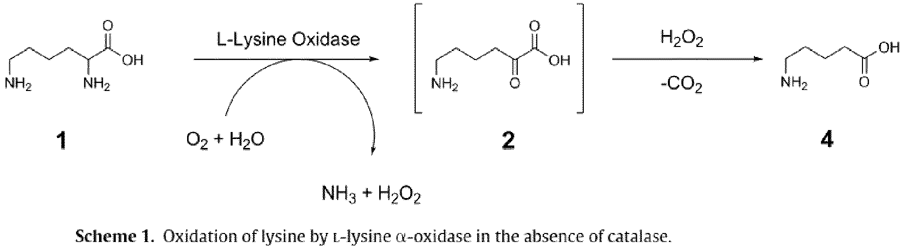

This process can be performed in an *ex vivo* system, wherein enzymes are immobilized in a support and lysine is added to be converted.

5-aminopentanoate can be polymerized into polyamide blocks (PA) or polyether block amide (PEBA) as it is well known by the man skilled in the art (as described in US 7,968,655 or in US 5,703,177 hereby incorporated by reference). PA blocks can be polymerized into homopolyamide or co-polyamide, containing 5-aminopentanoate and at least one another amino acid chosen for example from: aminocaproic acid, 7-amino heptanoic acid, 11-aminoundecanoic acid, and 12-aminododecanoic acid.

These polymers are used in particular to manufacture, wholly or in part, shaped articles, such as fibers, fabrics, films, sheets, rods, tubes or injection-moulded parts.

### EXAMPLES

The present invention is further defined in the following examples. It should be understood that these example, while indicating preferred embodiments of the invention, are given by way of illustration only. From above disclosure and these examples, the man skilled in the art can make various changes of the invention to adapt it to various uses and conditions without modifying the essentials means of the invention.

In particular, examples show modified *S. cerevisiae* or *Escherichia coli* (*E. coli*) strains, but these modifications can easily be performed in other microorganisms of the same family.

*Escherichia coli* belongs to the *Enterobacteriaceae* family, which comprises members that are Gram-negative, rod-shaped, non-spore forming and are typically 1-5 µm in length. Most members have flagella used to move about, but a few genera are non-motile. Many members of this family are a normal part of the gut flora found in the intestines of humans and other animals, while others are found in water or soil, or are parasites on a variety of different animals and plants. *E. coli* is one of the most important model organisms, but other important members of the *Enterobacteriaceae* family include *Klebsiella,* in particular *Klebsiella terrigena, Klebsiella planticola* or *Klebsiella oxytoca,* and *Salmonella.*

*Saccharomyces cerevisiae* is a model organism and belongs to the *Saccharomycetaceae* family, which comprises members that are spherical, elliptical or oblong acuminate cells with an asexual reproduction by budding and are typically 5-10 µm in diameter. Many members are used for industrial production due to their fermentative capacity. *S. cerevisiae* is one of the most important model organisms, but other important members of the *Saccharomycetaceae* family include *Pichia, Candida* and *Kluyveromyces,* in particular *Kluyveromyces lactis, Kluyveromyces marxianus* and *Pichia angusta.*

## Claims

1. Method for the fermentative production of 5-aminopentanoate from lysine or one precursor of lysine, comprising culturing a recombinant microorganism in a culture medium comprising a source of carbon and nitrogen, wherein in said microorganism, the 5-aminopentanoate biosynthesis pathway is composed of:
- The initial substrate 2-oxoadipate is converted into 5-aminopentanoate by the successive actions of an amino acid transaminase and an amino acid decarboxylase (pathway n°9); or
- The initial substrate 2-oxoadipate is converted into 5-aminopentanoate by the successive actions of an alpha keto acid decarboxylase and a transaminase (pathway n°10), or
- The initial substrate lysine is converted into 5-aminopentanoate by the successive actions of a transaminase, a keto acid coA ligase and a thioesterase (pathways n°5, 6, 7), or
- The initial substrate lysine is converted into 5-aminopentanoate by the successive actions of at least a transferase, a N6-acetyl-beta-lysine-transaminase and a 4-acetamidobutyrate deacetylase (pathway n°4).

2. Method according to claim 1, wherein at least one of the enzymes involved in the biosynthesis pathway is an evolved enzyme.

3. Method according to claim 1, wherein the 5-aminopentanoate biosynthesis pathway comprises the following successive steps:
- Conversion of 2-oxoadipate into L-2-aminoadipate by the catalytic action of an amino acid transaminase;
- Conversion of said L-2-aminoadipate into 5-aminopentanoate by the catalytic action of an evolved amino acid decarboxylase.

4. Method according to claim 3, wherein the amino acid transaminase is encoded by the gene ARO8 from Saccharomyces cerevisiae.

5. Method according to claim 3, wherein the evolved amino acid decarboxylase is encoded by a mutated gene *GAD1.*

6. Method according to claim 1, wherein the 5-aminopentanoate biosynthesis pathway comprises the following successive steps:
- Conversion of 2-oxoadipate into 5-oxopentanoate by the catalytic action of an evolved alpha-keto acid decarboxylase;
- Conversion of said 5-oxopentanoate into 5-aminopentanoate by the catalytic action of a transaminase.

7. Method according to claim 6, wherein the evolved alpha-keto acid decarboxylase is encoded by a mutated *sucA* gene.

8. Method according to claim 6, wherein the 5-aminovalerate transaminase is encoded by the gene *gabT* from *P. putida.*

9. Method according to claim 1, wherein the 5-aminopentanoate biosynthesis pathway comprises the following successive steps:
- Conversion of lysine into 6-amino-2-oxohexanoate by catalytic action of a transaminase,
- Conversion of 6-amino-2-oxohexanoate into 5-aminopentanoyl-coA by catalytic action of a ketoacid coA ligase,
- Conversion of 5-aminopentanoyl-coA into 5-aminopentanoate by catalytic action of a thioesterase.

10. Method according to claim 1, wherein the 5-aminopentanoate biosynthesis pathway comprises the following successive steps:
- Conversion of lysine into N6-acetyl-L-lysine by the catalytic action of a transferase,
- Conversion of N6-acetyl-L-lysine into 6-acetamido-2-oxohexanoate by the catalytic action of a N6-acetyl-beta-lysine transaminase,
- Conversion of 6-acetamido-2-oxohexanoate into 5-acetamidopentanoate,
- Conversion of 5-acetamidopentanoate into 5-aminopentanoate by the catalytic action of a 4-acetamidobutyrate deacetylase.

11. Method for the fermentative production of 5-aminopentanoate according to anyone of claims 1 to 10, wherein the microorganism is from the species *Escherichia coli* or *Saccharomyces cerevisiae.*

12. Method for the fermentative production of 5-aminopentanoate according to anyone of claims 1 to 11, comprising a step of isolation of the 5-aminopentanoate from the culture medium.

13. A genetically modified microorganism for the fermentative production of 5-aminopentanoate as defined in anyone of claims 1 to 11.
